# EUROPEAN PATENT APPLICATION

(11) **EP 4 545 117 A1**
(43) Date of publication of application: **30.04.2025**
(21) Application number: 23819819.6
(22) Date of filing: 06.06.2023
(51) Int. Cl.: A61M 5/145

(54) **INJECTION DEVICE AND METHOD FOR MANUFACTURING ACTUATOR**

(30) Priority: 07.06.2022 JP 2022092096
(71) Applicant: Circulus Inc., Tokyo 113-0033 (JP)
(72) Inventor: UEKI, Jun, Yokohama city, Kanagawa 232-0065 (JP); NEMOTO, Shigeru, Tokyo 113-0033 (JP)
(74) Representative: Viering, Jentschura & Partner mbB Patent- und Rechtsanwälte
(86) International application number: PCT/JP2023/020917
(87) International publication number: WO 2023/238839

(57) **Abstract**

An injection device 100 includes a syringe holder 110 on which a syringe S filled with a liquid medicine is mounted, a presser 115 that pushes out the liquid medicine from the mounted syringe S, an actuator 130 having a case 170 that houses a drive mechanism moving the presser 115 forward or backward, and a first cover 141 and a second cover 142 that house the actuator 130, wherein at least one of the first cover 141 or the second cover 142 is fixed to the case 170.

## Description

### Technical Field

The present invention relates to an injection device including an actuator having a case, and a method for manufacturing the actuator.

### Background Art

Patent Literature 1 discloses an injection device including a syringe holder on which a syringe filled with a liquid medicine is mounted, a presser that pushes out the liquid medicine from the mounted syringe, and an actuator. The actuator moves the presser forward or backward. For such a purpose, the actuator includes a feed screw nut, a feed screw shaft, a motor, and a transmission mechanism that transmits rotation from the motor to the feed screw shaft. Then, the feed screw nut, the feed screw shaft, the motor, and the transmission mechanism are housed in a case of the actuator. In addition, the injection device includes an upper cover and a lower cover that house the actuator.

In the injection device, the lower cover engages with the upper cover, and the lower cover and the upper cover are fixed to each other in a state where the actuator is received. Specifically, the upper cover is put on the syringe holder, and the lower cover is screwed to the upper cover so as to house the actuator. As a result, the weight of the lower cover and the upper cover are supported by a connection unit connected to a caster stand via the syringe holder and a connecting panel.

### Citation List

### Patent Literature

Patent Literature 1: JP6232169B

### Summary of Invention

### Technical Problem

In the injection device described in Patent Literature 1, when the weight of the upper cover increases, it is necessary to provide a frame to support the weight in a housing space formed inside the lower cover and the upper cover. For example, an injection device of a type in which two syringes are mounted has a heavier upper cover than an injection device of a type in which one syringe is mounted. However, if the frame is interposed, room for the housing space is narrowed. Therefore, it is necessary to increase sizes of the lower cover and the upper cover in order to house devices or parts inside the injection device. As a result, the size of the injection device increases, and the weight of the injection device increases.

### Solution to Problem

An injection device according to one mode described below comprises: a syringe holder on which a syringe filled with a liquid medicine is mounted; a presser that pushes out the liquid medicine from the mounted syringe; an actuator including a case that houses a drive mechanism moving the presser forward or backward; and a first cover and a second cover that house the actuator, wherein at least one of the first cover or the second cover is fixed to the case.

In addition, an injection device according to another mode is an injection device that injects a liquid medicine, and comprises: a presser that pushes out the liquid medicine; and an actuator including a drive mechanism that moves the presser forward or backward and a case that houses the drive mechanism, wherein the drive mechanism includes a feed screw shaft and a sliding member that moves along the feed screw shaft with rotation of the feed screw shaft, the sliding member includes a plurality of detents, a guide groove guiding each of the detents is formed in the case, and each of the detents is arranged in a groove portion formed in the sliding member and is movable in a radial direction of the feed screw shaft with respect to the groove portion.

Furthermore, a method for manufacturing an actuator according to still another mode is a method for manufacturing an actuator, the actuator including a drive mechanism including a feed screw shaft and a sliding member that moves along the feed screw shaft with rotation of the feed screw shaft, and a case that houses the drive mechanism, the sliding member including a plurality of detents, the method comprising: preparing the case in which a guide groove guiding each of the detents is formed; preparing the sliding member in which a groove portion is formed; applying a lubricant to at least either each of the detents or the groove portion; arranging each of the detents in the groove portion; and housing the drive mechanism inside the case in such a manner that each of the detents is located between the guide groove and the groove portion.

Accordingly, the size of the injection device can be reduced, and the weight of the injection device can be reduced.

### Brief Description of Drawings

FIG. 1 is a schematic perspective view of an injection device as viewed from above.
FIG. 2 is a schematic perspective view of the inside of the injection device as viewed from below.
FIG. 3 is a schematic perspective view of the inside of the injection device as viewed from above.
FIG. 4 is a schematic perspective view of an actuator viewed from above.
FIG. 5 is a schematic perspective view of the actuator and a rear unit as viewed from below.
FIG. 6 is a schematic perspective view of a front unit as viewed from below.
FIG. 7 is a schematic perspective view of a device support as viewed from below.
FIG. 8 is a schematic exploded view of a part of the injection device.
FIG. 9 is a schematic perspective view of a coupling portion according to a second embodiment as viewed from above.
FIG. 10 is a schematic exploded perspective view of the actuator.
FIG. 11 is a schematic perspective view of a ball screw nut.
FIG. 12 is a schematic perspective view of a detent.
FIG. 13 is a schematic cross-sectional view of the detent.
FIG. 14A illustrates the detent before movement, and FIG. 14B illustrates the detent after movement.
FIG. 15A illustrates the detent that is not moving, and FIG. 15B illustrates a detent that is moving.

### Description of Embodiments

Hereinafter, exemplary embodiments for carrying out the present invention will be described in detail with reference to the drawings. Note that dimensions, materials, shapes, and relative positions of components described in the following embodiments can be arbitrarily set, and can be changed according to a configuration of a device or a method to which the present invention is applied or various conditions. In addition, unless otherwise specified, the scope of the present invention is not limited to the embodiments specifically described below. Incidentally, in the present specification, up and down correspond to the upward direction and the downward direction in the direction of gravity, respectively. In addition, in the present specification, the front side ("front") corresponds to a distal end portion side of a syringe, and the opposite side corresponds to the rear side ("rear").

### [First Embodiment]

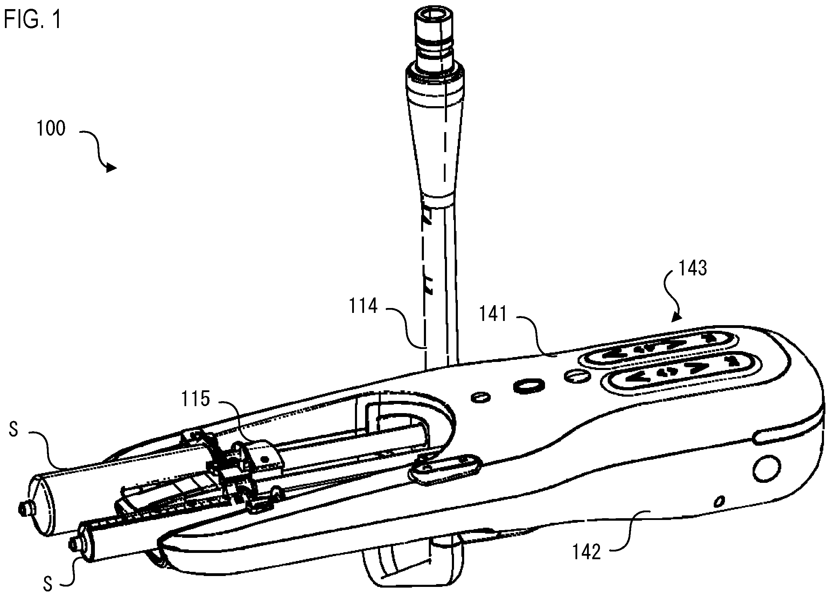

An injection device 100 for injecting a liquid medicine will be described with reference to FIGS. 1 to 3. The injection device 100 includes syringe holders 110 on which syringes S each of which is filled with the liquid medicine are mounted, respectively. In the injection device 100 illustrated in FIG. 1, two syringes S can be mounted, and each of the two syringes S is filled with, for example, a contrast medium or physiological saline. In addition, the injection device 100 includes pressers 115 that push out the liquid medicines from the mounted syringes S, respectively. Specifically, the presser 115 pushes out rear end of the mounted syringe S, that is, the rear end of piston in the syringe S.

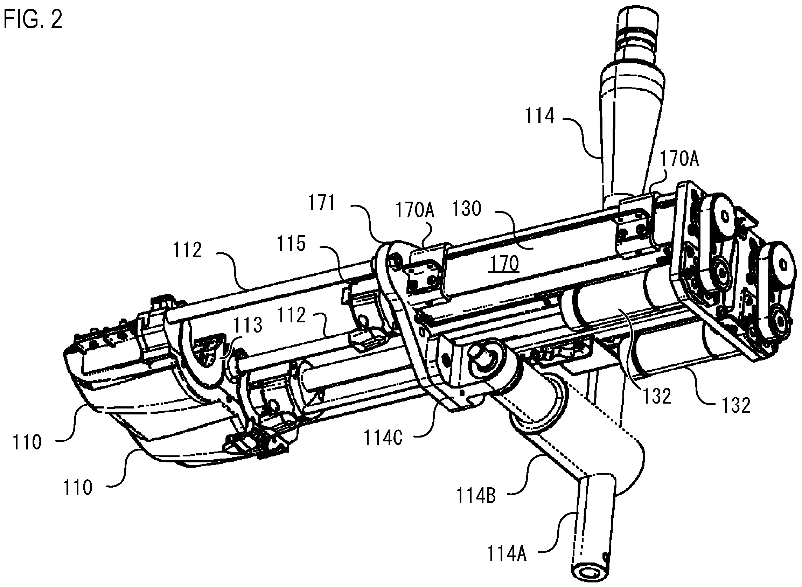

Furthermore, the injection device 100 includes actuators 130 (FIG. 2) having drive mechanisms that move the pressers 115 forward or backward, respectively. Then, the injection device 100 includes a first cover and a second cover that house the actuators 130, and an example of the first cover is an upper cover 141 and an example of the second cover is a lower cover 142.

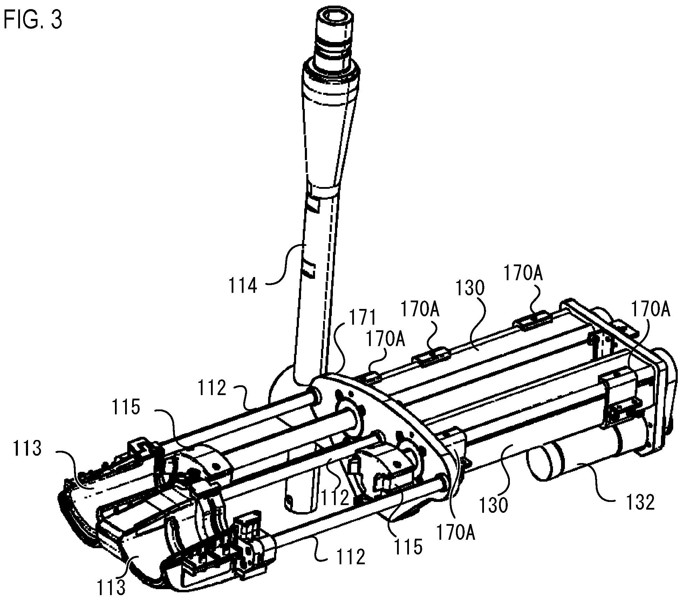

FIG. 1 is an upper perspective view when the injection device 100 in a state in which the syringes S are mounted is viewed from the front. In addition, FIG. 2 is a lower perspective view when the inside of the injection device 100 in a state where the syringes S are not mounted is viewed from the rear. Then, FIG. 3 is an upper perspective view when the inside of the injection device 100 in the state where the syringes S are not mounted is viewed from the front. Furthermore, FIGS. 2 and 3 illustrate the injection device 100 from which the upper cover 141 and the lower cover 142 are detached.

The syringe holders 110 illustrated in FIG. 2 are supported by holder supports 112. In addition, the plurality of syringe holders 110 is integrally formed. However, each of the syringe holders 110 may be a separate body. Then, the holder support 112 is fixed to the actuator 130 via a front plate 171. The holder supports 112 are located on both sides of the presser 115 and extend along a traveling direction of the presser 115. In addition, the syringe holder 110 has a recess 113 for receiving the syringe S, and the mounted syringe S is fixed to the syringe holder 110. Incidentally, the piston slidable inside the syringe S is attached to the syringe S mounted on the injection device 100. Alternatively, the syringe holder 110 may be supported by the front plate 171. In addition, the syringe S may be held by the front plate 171.

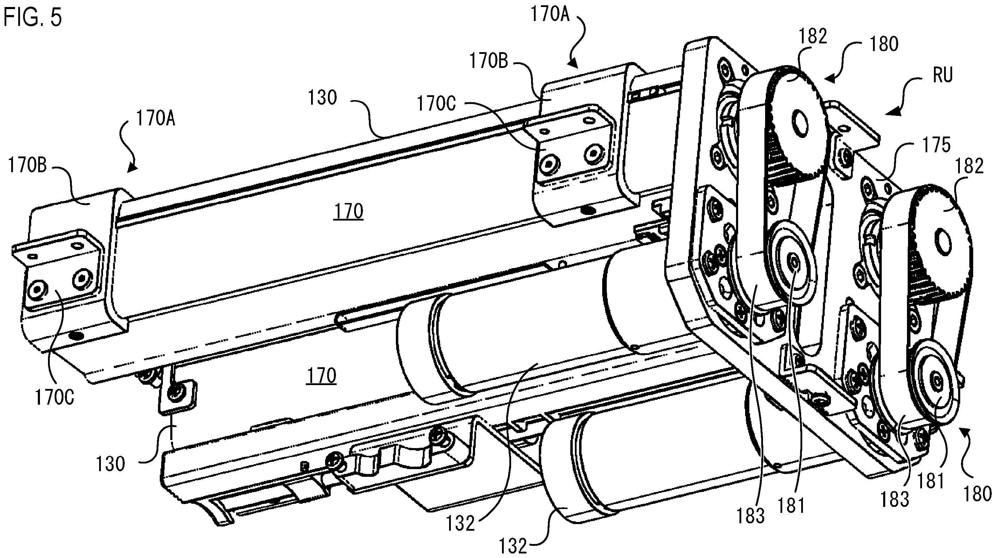

The actuator 130 includes a feed screw nut and a feed screw shaft as examples of the drive mechanism which is not illustrated. Then, the feed screw shaft is connected to driven gear 182 (FIG. 5) of transmission mechanism 180. Accordingly, the transmission mechanism 180 transmits rotation from a motor 132 to the actuator 130 (specifically, the feed screw shaft). Then, when the motor 132 rotates forward in a state where the presser 115 abuts on the rear end of the piston, the presser 115 pushes the piston in the front direction.

Accordingly, the piston moves forward, so that the liquid medicine in the syringe S is pushed out. Then, the pushed-out liquid medicine is injected into a body of a patient via an extension tube connected to a distal end of the syringe S, a mixing device, and the like. On the other hand, when the motor 132 rotates in the reverse direction, the presser 115 pulls the piston in the rear direction, and the liquid medicine is sucked into the syringe S. Incidentally, a claw provided in the presser 115 can be omitted. If the claw is omitted or the syringe S is removed, only the presser 115 moves backward when the motor 132 rotates in the reverse direction .

As illustrated in FIG. 1, the upper cover 141 is provided with operation buttons 143 such as forward buttons, backward buttons, and start buttons. In addition, a substrate which is not illustrated is attached to the upper cover 141 on a side facing the actuator 130, and the operation buttons 143 are connected to the substrate. Then, an operator can manually operate the injection device 100 by operating the operation buttons 143. Specifically, the pressers 115 move forward while the operator presses the forward buttons. Furthermore, the pressers 115 move backward while the operator presses the backward buttons. In addition, the injection device 100 starts to inject the liquid medicines when the operator presses the start buttons.

The lower cover 142 is configured to engage with the upper cover 141. Then, in a state where the actuators 130 are received, the lower cover 142 and the upper cover 141 are fixed to each other by a method such as screwing. Accordingly, the actuators 130 are housed between the lower cover 142 and the upper cover 141. Incidentally, at least one of the upper cover 141 and the lower cover 142 may be configured by assembling a plurality of parts. For example, at least one of the upper cover 141 and the lower cover 142 may be configured by a front part and a rear part that are separated from each other.

As illustrated in FIG. 2, the injection device 100 has a device support 114 connected to a caster stand. The device support 114 is connected to the front plate 171 via a connection member 114C having a substantially T-shaped cross section. Furthermore, the device support 114 extends laterally of the injection device 100 and is exposed from the lower cover 142 and the upper cover 141. In addition, the front plate 171 is fixed to front faces of the actuators 130.

A pole portion 114A of the device support 114 is connected to the caster stand placed on a floor surface. Accordingly, the injection device 100 is rotatably supported by the device support 114 and the caster stand. For example, the injection device 100 can rotate to a posture in which the front side (that is, side on which the syringe S is mounted) of the injection device 100 faces the floor surface and a posture in which the rear side (that is, side opposite to the side on which the syringe S is mounted) of the injection device 100 faces the floor surface. In addition, the injection device 100 is preferably connected to the caster stand so as to be rotatable in the left-right direction. Incidentally, the device support 114 can also be connected to a ceiling suspension member installed on a ceiling. Then, when the device support 114 is connected, the injection device 100 is supported by the device support 114 and the ceiling suspension member.

In addition, the injection device 100 is connected to a control device which is not illustrated in a wired or wireless manner. The control device includes a touch panel and functions as a controller of the injection device 100. In the control device, operation pattern data, liquid medicine data, and the like are stored in advance. Then, when the liquid medicines are injected into the patient, the operator operates the touch panel to input an injection rate, an injection amount, an injection time, and physical data of the patient such as a body weight, and the liquid medicine data such as an iodine amount and types of the liquid medicines to the control device.

The control device calculates optimum injection conditions according to the input data and the data stored in advance. Then, the control device decides amounts of the liquid medicines to be injected into the patient and an injection protocol based on the calculated injection conditions. Thereafter, the injection device 100 injects the liquid medicines according to the decided injection protocol. Incidentally, the control device can also acquire the operation pattern data, other data, and the like from an external storage medium.

As illustrated in FIGS. 2 and 3, case 170 of the actuator 130 is provided with clamp members 170A as examples of a fixing portion to which at least one of the upper cover 141 and the lower cover 142 is fixed. In the example of FIGS. 2 and 3, the upper cover 141 is fixed to the clamp members 170A. In addition, two or three clamp members 170A are arranged side by side in the longitudinal direction of the case 170, that is, in the front-rear direction of the injection device 100. Alternatively, the number of the clamp members 170A may be one or four or more. Furthermore, each of a plurality of actuators 130 may be provided with the same number of the clamp members 170A.

When the plurality of actuators 130 is provided, each of the clamp members 170A is provided at a position avoiding a space between the plurality of actuators 130. For example, in FIGS. 2 and 3, each of the clamp members 170A is arranged on faces opposite to mutually facing faces of the two actuators 130. That is, each of the clamp members 170A is arranged while avoiding a space between the two actuators 130. As a result, a space inside the injection device 100 can be more efficiently used. For example, the substrate extending from the upper cover 141 can be arranged in the space between the two actuators 130.

Incidentally, if three or more actuators 130 are provided, each of the clamp members 170A can be arranged on faces opposite to the mutually facing faces of the two actuators 130 arranged to oppose. As a result, each of the clamp members 170A is arranged on lateral sides of the two outermost actuators 130. In other words, each of the clamp members 170A is arranged on the outermost faces of the actuators 130 in a direction orthogonal to the up-down direction, that is, arranged on the faces opposing the upper cover 141 or the lower cover 142.

### [Actuator]

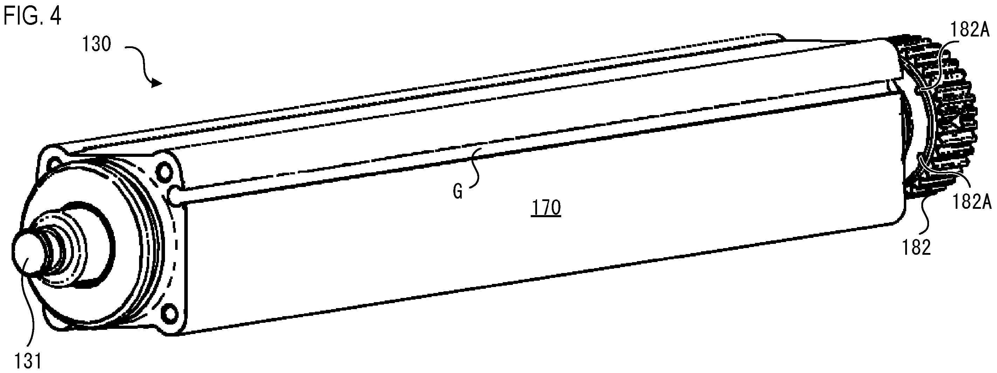

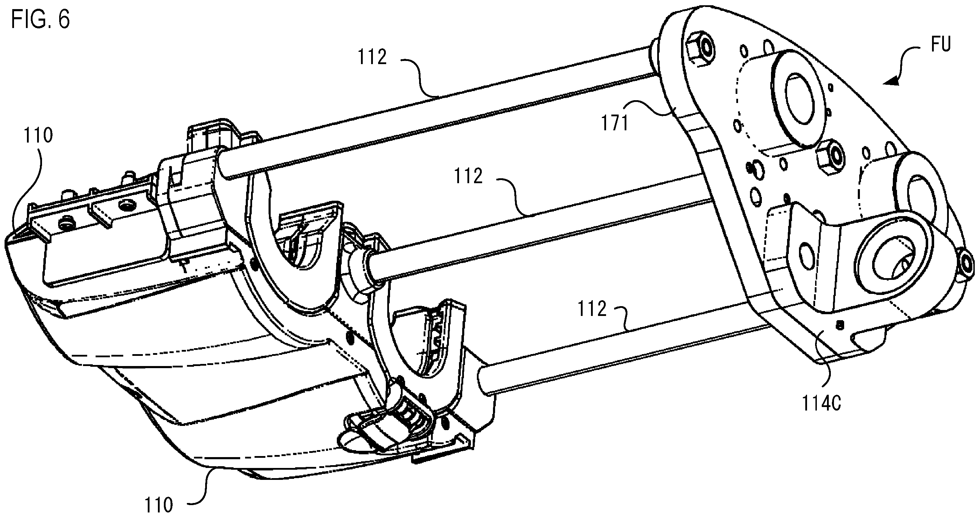

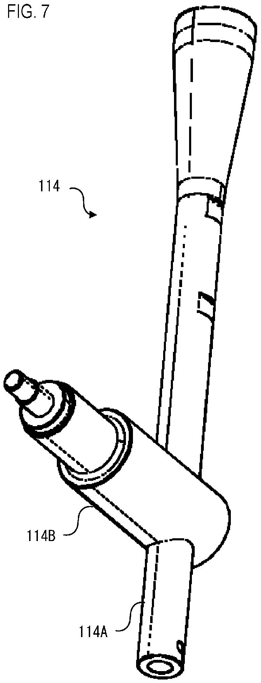

Subsequently, the actuator 130 will be described with reference to FIGS. 4 to 7. Incidentally, FIG. 4 illustrates an upper perspective view of the actuator 130 as viewed from the front. In addition, FIG. 5 is a lower perspective view of the actuators 130 and a rear unit RU fixed to the actuators 130 as viewed from the rear. Furthermore, FIG. 6 is a lower perspective view of a front unit FU to which the actuators 130 are fixed as viewed from the rear. In addition, FIG. 7 also illustrates a lower perspective view of the device support 114 as viewed from the rear.

The actuator 130 illustrated in FIG. 4 is connected to the driven gear 182 of the transmission mechanism 180 (FIG. 5) that transmits the rotation from the motor 132. Specifically, the actuator 130 includes a ball screw shaft (not illustrated) as the feed screw shaft connected to the driven gear 182 of the transmission mechanism 180. In addition, the actuator 130 includes a ball screw nut (not illustrated) as the feed screw nut attached to the ball screw shaft. The ball screw shaft and the ball screw nut are examples of the drive mechanism that moves the presser 115 forward or backward.

In addition, the actuator 130 includes the case 170 having a substantially prismatic shape. A space is formed inside the case 170. Then, the case 170 houses the ball screw nut and the ball screw shaft. Accordingly, the actuator 130 is modularized. In addition, the case 170 has rigidity enough to support at least a part of the weight of the upper cover 141. As an example, the case 170 is formed by extrusion of aluminum. Alternatively, the case 170 may be formed by die casting of aluminum. In addition, a plurality of guide grooves (described later) extending along the longitudinal direction of the case 170 is formed inside the case 170. Then, the ball screw nut is provided with a plurality of detents made of resin, and the detents are fitted in corresponding groove portions (described later), respectively. As an example, the number of the groove portions is five, but may be six or more or four or less.

A groove G extending along the longitudinal direction of the case 170 is formed on a side face of the case 170. A limit detector (not illustrated) that detects a limit position of movement of the ball screw nut can be arranged in the groove G. The limit position is set to correspond to a position that is allowed as a design limit by which the presser 115 can move forward or backward in design. In addition, the groove G is formed over the entire length of the case 170. Accordingly, a position of the limit detector can be changed along the longitudinal direction of the case 170. Then, a plurality of the limit detectors is arranged side by side along the ball screw shaft. For example, the limit detectors include a front limit detector and a rear limit detector. Furthermore, the limit detectors may include a central detector that detects a home position.

Furthermore, a magnet for limit detection is arranged in the ball screw nut. Then, the limit detectors detect the limit positions by detecting the magnetism of the magnet for limit detection. In addition, the actuator 130 is arranged such that the groove G on the side face of the case 170 is located on a side opposite to the motor 132. That is, the actuator 130 is arranged such that the groove G is located above. Accordingly, it is possible to suppress the limit detectors from being affected by a magnetic force generated from the motor 132.

Furthermore, the actuator 130 includes a presser pipe 131. Then, the presser pipe 131 is connected to the ball screw nut. The ball screw nut is attached to the ball screw shaft. In addition, the transmission mechanism 180 (FIG. 5) includes the driven gear 182 as an example of a rotation member that transmits the rotation from the motor 132 to the actuator 130. Then, the ball screw shaft is connected to the driven gear 182 and rotates with rotation of the driven gear 182.

In addition, magnets 182A for a magnetic encoder are arranged on the driven gear 182 on a side facing the actuator 130. As an example, a plurality of the magnets 182A is arranged, and the number thereof is six. However, the number of the magnets 182A may be five or less or seven or more. For example, the plurality of magnets 182A is buried at equal intervals so as to be arrayed on a circle concentric with the center of the driven gear 182 on a face on the side facing the actuator 130. Therefore, when the number of the magnets 182A is six, angles defined by lines connecting the magnets 182A and the center of the driven gear 182 are 60°. Alternatively, the plurality of magnets 182A may be attached to the driven gear 182 by an adhesive. In addition, adjacent magnets 182A among the plurality of magnets 182A have mutually opposite polarities.

The magnetic encoder (not illustrated) is arranged in a space between the actuator 130 and the driven gear 182. The magnetic encoder detects at least one of a rotational position, a rotational direction, and a rotational speed of the driven gear 182 as a change in a magnetic field. Then, the magnetic encoder converts information indicating at least one of the rotational position, the rotational direction, and the rotational speed into an electric signal and outputs the electric signal to the control device of the injection device 100. As an example, the magnetic encoder is attached to a face of the case 170 on a side facing the driven gear 182 by a method such as screwing. Accordingly, it is not necessary to separately provide room for providing the encoder, and an internal space of the injection device 100 can be more efficiently used.

The rear unit RU illustrated in FIG. 5 includes a rear plate 175 having a substantially rectangular outer shape. In addition, the rear unit RU includes the motors 132 fixed to the rear plate 175 and the transmission mechanisms 180 attached to the rear plate 175. As an example, the motor 132 is fixed to the rear plate 175 by a method such as screwing. Alternatively, the motor 132 may be fixed with the rear plate 175 by bolts and nuts, an adhesive, or the like.

The transmission mechanism 180 includes a driving gear 181 connected to a shaft of the motor 132 and the driven gear 182 connected to the ball screw shaft. That is, the driven gear 182 is located above the driving gear 181 in FIG. 5. Then, a timing belt 183 is wound around the driven gear 182 and the driving gear 181. As an example, the driving gear 181 and the driven gear 182 are made of metal, and can be formed using, for example, a steel material such as stainless steel.

Then, rotation of the shaft of the motor 132 is transmitted to the ball screw shaft of the actuator 130 via the transmission mechanism 180. Accordingly, the ball screw shaft rotates according to the transmitted rotation. As a result, the ball screw nut slides in the front direction or rear direction with rotation of the ball screw shaft. As the ball screw nut slides, the presser pipe 131 and the presser 115 connected to the presser pipe 131 move forward or backward.

The driven gear 182 is connected to the actuator 130, to which the rear plate 175 is fixed. In addition, the driving gear 181 is connected to the motor 132, which is fixed to the rear plate 175. Then, the timing belt 183 is wound around the driven gear 182 and the driving gear 181. Accordingly, the transmission mechanism 180 is attached to the rear plate 175.

In addition, the rear unit RU is fixed to the cases 170 of the actuators 130 via the rear plate 175 in a detachable manner. As an example, the rear plate 175 is fixed to the cases 170 by a method such as screwing. Accordingly, the cases 170 can support at least a part of the weight of the rear unit RU. That is, the cases 170 can support the weight of the transmission mechanisms 180 and the motors 132 via the rear plate 175. Alternatively, the rear plate 175 may be fixed to the cases 170 by bolts and nuts.

Each of the cases 170 of the plurality of actuators 130 is coupled by the one rear plate 175 which is an example of a plate member. Specifically, the rear plate 175 is fixed to the plurality of cases 170 in a detachable manner. Accordingly, when one of the actuators 130 is driven, it is possible to suppress the actuator 130 from being slightly displaced due to a reaction force. Alternatively, the rear plate 175 corresponding to each of the actuators 130 may be provided. In such a case, a plurality of the rear plates 175 can be detached from the cases 170, respectively. Therefore, when any one of the plurality of actuators 130 is to be replaced, replacement work can be performed by detaching only the rear plate 175 fixed to the actuator 130 to be replaced.

In addition, at least one of the upper cover 141 and the lower cover 142 may be directly or indirectly fixed to the rear plate 175. In such a case, the rear plate 175 functions as an example of the fixing portion to which at least one of the upper cover 141 and the lower cover 142 is fixed. Accordingly, the cases 170 can support the weight of at least one of the upper cover 141 and the lower cover 142 via the rear plate 175.

In addition, as illustrated in FIG. 5, the clamp member 170A includes a main body portion 170B having a substantially U-shaped cross section and a support fitting 170C that has a substantially L-shape and is fixed to the main body portion 170B. The main body portion 170B is bent in a substantially U shape, and the case 170 is inserted inside the main body portion 170B. In addition, the main body portion 170B is fixed to the case 170 by screwing. Then, the support fitting 170C is fixed to the main body portion 170B and the case 170 by screwing. Alternatively, the main body portion 170B and the support fitting 170C may be fixed by a bolt and a nut. Furthermore, the main body portion 170B and the support fitting 170C may be integrally formed.

Then, at least one of the upper cover 141 and the lower cover 142 is fixed to the cases 170. In the example of FIG. 5, the upper cover 141 is fixed to upper faces of the support fittings 170C by screwing. Alternatively, the upper cover 141 may be fixed by bolts and nuts. Incidentally, the clamp member 170A only needs to have the fixing portion to which at least one of the upper cover 141 and the lower cover 142 is fixed, and a shape thereof can be appropriately changed. For example, at least one of the upper cover 141 and the lower cover 142 may be fixed to an upper face or side faces of the main body portion 170B, and the main body portion 170B may function as the fixing portion. In such a case, the support fitting 170C can be omitted.

In addition, the main body portion 170B functioning as the fixing portion may have another shape such as a substantially T-shaped cross section or a substantially L-shaped cross section. Furthermore, the lower cover 142 may be fixed to the clamp members 170A. In such a case, to the clamp members 170A, only the lower cover 142 may be fixed, or the upper cover 141 and the lower cover 142 may be fixed.

Incidentally, the upper cover 141 and the lower cover 142 may be fixed to the cases 170 directly or indirectly via another member. For example, the upper cover 141 may be directly fixed to upper faces or side faces of the cases 170. Furthermore, the lower cover 142 may be directly fixed to lower faces or the side faces of the cases 170. In addition, the fixing portion may be formed integrally with the case 170 instead of the clamp member 170A which is separate body from the case 170. For example, the fixing portion may be a flange portion protruding laterally from the case 170.

The front unit FU illustrated in FIG. 6 includes the front plate 171 having a substantially triangular outer shape. In addition, the front unit FU includes the syringe holders 110 supported by the holder supports 112. As an example, the holder support 112 is fixed to the front plate 171 by a method such as screwing. Alternatively, the holder support 112 may be fixed to the front plate 171 by bolts and nuts. In addition, the actuator 130 is fixed to the front plate 171 in a detachable manner. For example, the case 170 of the actuator 130 is fixed to the front plate 171 by a method such as screwing. Alternatively, the case 170 may be fixed to the front plate 171 by bolts and nuts.

Furthermore, the front plate 171 is connected to the device support 114 via the connection member 114C. Alternatively, the front plate 171 may be connected to the device support 114 via the cases 170 of the actuators 130. In such a case, the cases 170 can support the weight of the syringe holders 110, the holder supports 112, and the syringes S, which are mounted on the syringe holders 110, via the front plate 171.

The device support 114 illustrated in FIG. 7 has the pole portion 114A extending in the up-down direction and a shaft portion 114B extending in the direction orthogonal to the up-down direction. Then, a distal end of the shaft portion 114B is inserted into an insertion opening having a substantially circular shape and formed in the connection member 114C, and is connected to the connection member 114C. In addition, a display can be attached to the pole portion 114A. The display displays information regarding the operation pattern (for example, injection protocol) of the injection device 100, patient information, information regarding the liquid medicines, and the like.

### [Assembly Method]

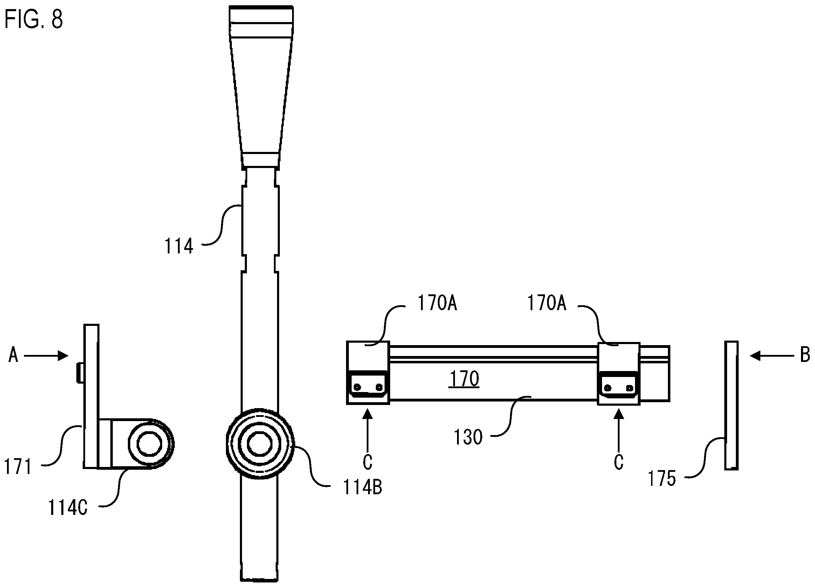

An assembly method of the injection device 100 will be described with reference to FIG. 8. Incidentally, FIG. 8 is a schematic exploded view of the front plate 171, the device support 114, the actuators 130, and the rear plate 175 as viewed from the side opposite to the device support 114. For convenience of description, the front unit FU, the rear unit RU, and other devices or members are not illustrated.

First, the syringe holders 110, the holder supports 112, and the like are attached to the front plate 171 to assemble the front unit FU. In addition, the motors 132 and the like are attached to the rear plate 175 to assemble the rear unit RU. Then, the actuators 130 prepared in advance are fixed to the front plate 171. For example, the cases 170 of the actuators 130 are screwed to the front plate 171 by screws threaded from a screwing direction A.

Furthermore, the rear plate 175 is fixed to the actuators 130. For example, the rear plate 175 is screwed to the cases 170 by screws threaded from a screwing direction B. Incidentally, screwing positions of the rear plate 175 with respect to the cases 170 are located on the same straight lines as screwing positions of the cases 170 with respect to the front plate 171, respectively. Specifically, screw holes located on the same straight lines are formed at four corners of a front face and a rear face of each of the cases 170. Then, the front plate 171 and the rear plate 175 are fixed to each of the cases 170 by threading screws into the screw holes.

Subsequently, the transmission mechanisms 180 and the like are attached to the rear plate 175. Thereafter, an assembly including the front unit FU, the actuators 130, and the rear unit RU is attached to the device support 114. Specifically, the front plate 171 is connected to the shaft portion 114B of the device support 114 via the connection member 114C. Thereafter, the upper cover 141 to which the substrate is attached is fixed to the cases 170 of the actuators 130. Specifically, the upper cover 141 is fixed to the clamp members 170A fixed to the cases 170 by a method such as screwing.

For example, the upper cover 141 is screwed to the clamp members 170A by screws threaded from a screwing direction C. Alternatively, the upper cover 141 may be fixed to the cases 170 by bolts and nuts. Then, the lower cover 142 is attached to the upper cover 141. As an example, the lower cover 142 is attached to the upper cover 141 by a method such as screwing. For example, the lower cover 142 is screwed to the upper cover 141 by screws threaded from a direction opposite to the screwing direction C. Accordingly, the front unit FU, the actuators 130, and the rear unit RU are housed in the space between the upper cover 141 and the lower cover 142. As described above, the injection device 100 is assembled.

In addition, when the actuator 130 is to be replaced, first, the lower cover 142 is detached from the upper cover 141. Subsequently, the upper cover 141 is detached from the actuators 130. Then, the rear plate 175 of the rear unit RU is detached from the actuators 130. Furthermore, the actuator 130 is detached from the front plate 171 of the front unit FU. Accordingly, the actuator 130 can be detached from the injection device 100 and replaced.

With the injection device 100 according to the first embodiment described above, the weight of devices or parts constituting the injection device 100 can be supported by the cases 170 of the actuators 130. Therefore, a frame for supporting the weight can be omitted, and the internal space of the injection device 100 can be suppressed from being narrowed. As a result, the size of the injection device 100 can be reduced, and the weight of the injection device 100 can be reduced. Furthermore, even a failure occurs in the actuators 130, replacement at an installation place of the injection device 100 can be performed.

Incidentally, the injection device 100 includes the two syringe holders 110 in the first embodiment. However, the injection device 100 may include one syringe holder 110 or three or more syringe holders 110. In such a case, the injection device 100 includes the actuators 130 and the pressers 115 in a number corresponding to the number of the syringe holders 110.

### [Second Embodiment]

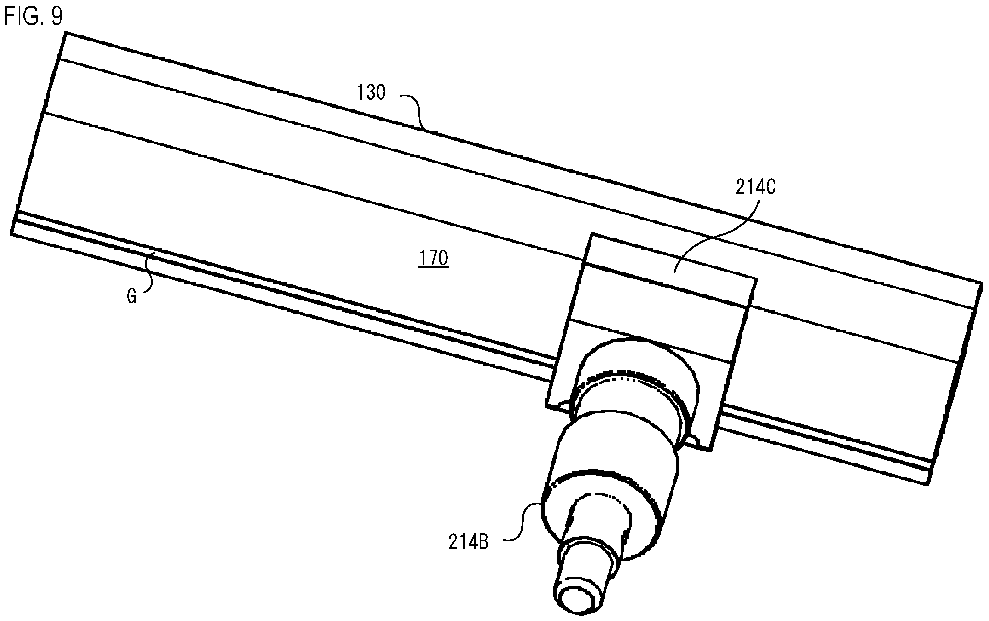

A second embodiment will be described with reference to FIG. 9. The second embodiment is different from the first embodiment in terms of further including a coupling portion that couples the device support 114 of the injection device 100 to the case 170. Specifically, in the injection device 100 of the second embodiment, a shaft portion 214B of the device support 114 is fixed to a connection member 214C which is an example of the coupling portion.

FIG. 9 illustrates an upper perspective view of the actuator 130, the shaft portion 214B, and the connection member 214C as viewed from the front. Incidentally, illustration of other devices and members are omitted for convenience of description. In the following description of the second embodiment, differences from the first embodiment will be described, and the already described components are denoted by the same reference signs, and the description thereof will be omitted. Unless otherwise described, the components denoted by the same reference signs exhibit substantially the same operation and function, and functions and effects thereof are also substantially the same.

The shaft portion 214B extends from the device support 114, which is not illustrated, in the direction orthogonal to the up-down direction. Then, the distal end of the shaft portion 214B is inserted into an insertion opening having a substantially circular shape and formed in the connection member 214C, which is an example of the coupling portion, and is connected to the connection member 214C. In addition, the connection member 214C is fixed to the case 170 of the actuator 130. The connection member 214C has a substantially U-shaped cross section, and the case 170 is inserted inside the connection member 214C. Then, the connection member 214C is fixed to the case 170 by screwing. Alternatively, the connection member 214C may be fixed by a bolt and a nut.

In addition, the connection member 214C may also function as a fixing portion to which the upper cover 141 is fixed. For example, the upper cover 141 may be fixed to an upper face or a side face of the connection member 214C. Furthermore, the connection member 214C may also function as a fixing portion to which the lower cover 142 is fixed. For example, the lower cover 142 may be fixed to a lower face or the side face of the connection member 214C. In addition, the connection member 214C may be formed integrally with the case 170 of the actuator 130. For example, the connection member 214C may be a flange portion protruding laterally from the case 170.

The front unit FU and the rear unit RU, which are not illustrated, are fixed to the actuator 130 in a detachable manner. For example, the actuator 130 is inserted into the connection member 214C and attached to the connection member 214C. Thereafter, the front plate 171 and the rear plate 175 are fixed to the case 170 by a method such as screwing. Accordingly, the front unit FU and the rear unit RU can be detached from the actuator 130. Alternatively, the front plate 171 and the rear plate 175 may be fixed to the case 170 by bolts and nuts.

In the second embodiment, the connection member 214C connected to the device support 114 via the shaft portion 214B is fixed to the case 170. Therefore, the weight of the injection device 100 is applied to the device support 114 via the case 170. That is, the case 170 can support the weight of the front unit FU and the rear unit RU and the weight of the upper cover 141 and the lower cover 142.

With the injection device 100 according to the second embodiment described above, the weight of devices or parts constituting the injection device 100 can be supported by the case 170 of the actuator 130. Therefore, a frame for supporting the weight can be omitted, and an internal space of the injection device 100 can be suppressed from being narrowed. As a result, the size of the injection device 100 can be reduced, and the weight of the injection device 100 can be reduced. Furthermore, even a failure occurs in the actuator 130, replacement at an installation place of the injection device 100 can be performed.

### [Detent]

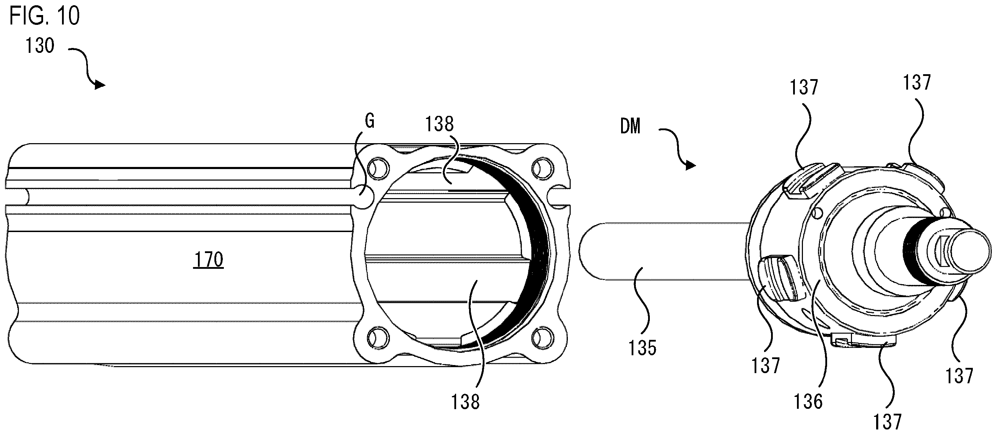

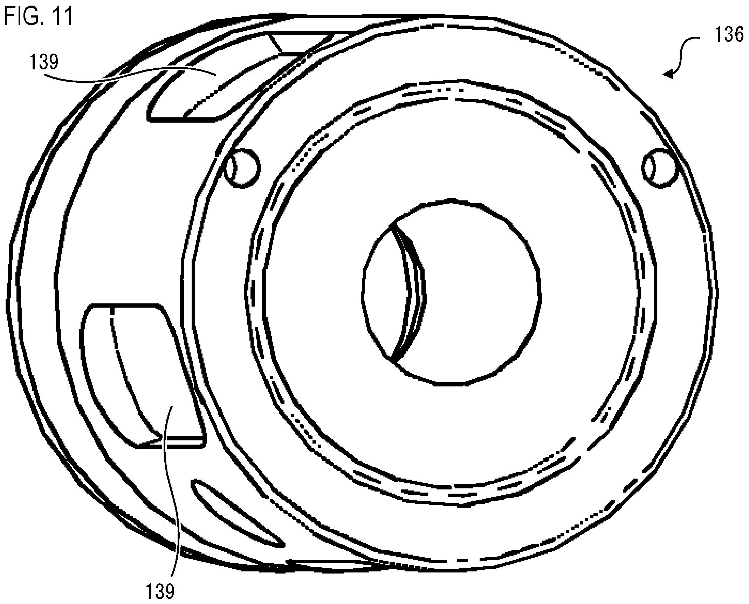

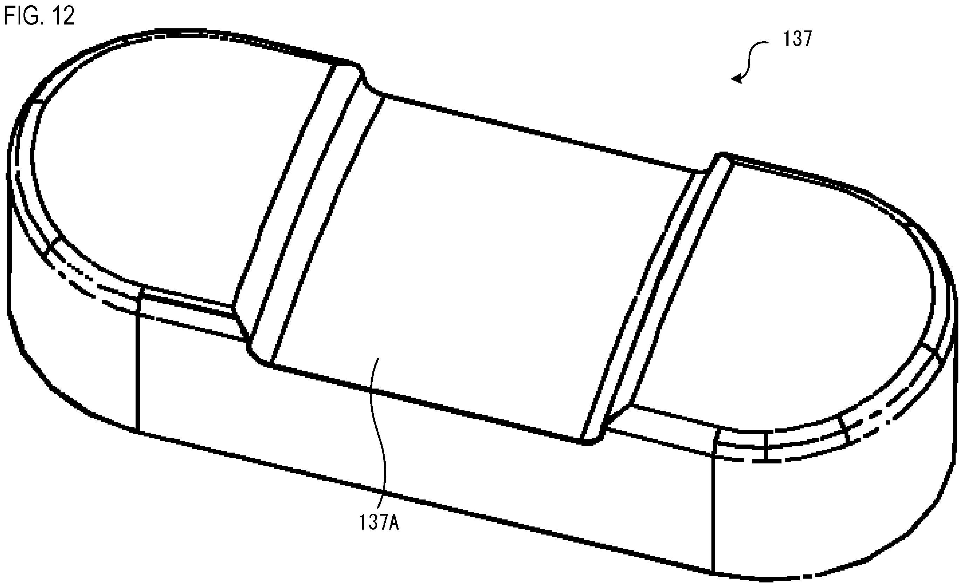

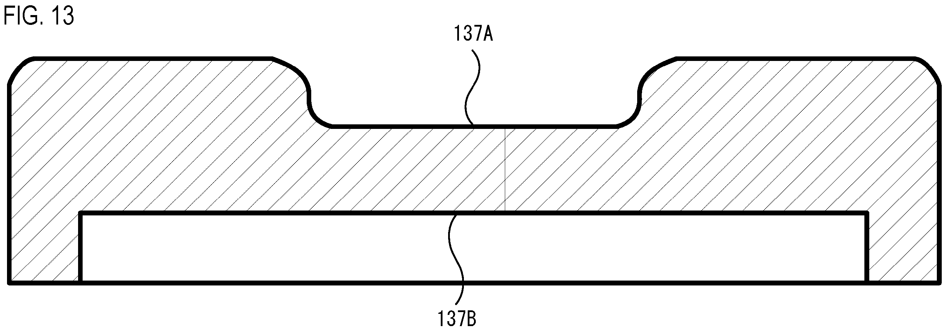

A detent 137 provided in the actuator 130 of the injection device 100 according to the first and second embodiments will be described with reference to FIGS. 10 to 13. The detent 137 is attached to a ball screw nut 136 of a drive mechanism DM included in the actuator 130. Incidentally, FIG. 10 is an exploded perspective view of the actuator 130 as viewed from the rear. In addition, FIG. 11 is a perspective view of the ball screw nut 136 before attachment of the detent 137. In addition, FIG. 12 is a perspective view of the detent 137, and FIG. 13 is a cross-sectional view along the longitudinal direction of a central portion of the detent 137.

As illustrated in FIG. 10, the actuator 130 includes the case 170. Then, the case 170 houses the drive mechanism DM that moves the presser 115 forward or backward. The drive mechanism DM illustrated in FIG. 10 includes a ball screw shaft 135 as an example of the feed screw shaft and the ball screw nut 136 which is the feed screw nut as an example of a sliding member. Then, the ball screw nut 136 is attached to the ball screw shaft 135. In addition, the ball screw nut 136 is connected to the presser pipe 131 (FIG. 4). The ball screw shaft 135 is inserted into the presser pipe 131. Then, the ball screw shaft 135 is connected to the driven gear 182 (FIG. 5) of the transmission mechanism 180. Incidentally, in FIG. 10, illustrations of members such as the presser pipe 131 are omitted.

In addition, the ball screw nut 136 includes a circulation portion where balls circulate and an outer cylinder portion that houses the circulation portion. Alternatively, the ball screw nut 136 according to another example of the sliding member does not necessarily have the outer cylinder portion. Furthermore, the ball screw nut 136 has a plurality of the detents 137. As an example, the detents 137 are arranged at equal intervals on an outer peripheral face of the ball screw nut 136. Then, the ball screw nut 136 moves in the front or rear direction along the ball screw shaft 135 as the ball screw shaft 135 rotates. In addition, guide grooves 138 that guide the detents 137, respectively, are formed in the case 170. As an example, the guide grooves 138 are formed when the case 170 is formed by extrusion. Alternatively, the guide groove 138 may be formed by cutting, pressing, or the like.

The guide groove 138 extend along the ball screw shaft 135 in the longitudinal direction of the case 170 and have a substantially U-shaped cross section. As an example, five guide grooves 138 are formed in the case 170. In addition, the ball screw nut 136 has five detents 137. Then, each of the detents 137 is fitted inside the guide groove 138, when the ball screw shaft 135 and the ball screw nut 136 are housed in the case 170. The detent 137 slides inside the guide groove 138, along with the movement of the ball screw nut 136. Alternatively, the number of the detents 137 may be four or less or six or more. In such a case, the number of the guide grooves 138 is the same as the number of the detents 137.

As illustrated in FIG. 11, a plurality of groove portions 139 is formed on the outer peripheral face of the outer cylinder portion of the ball screw nut 136. As an example, the number of the groove portions 139 is five, which is the same as the number of the detents 137. Then, the detent 137 is arranged in the groove portion 139, formed in the ball screw nut 136. Furthermore, the detent 137 is movable with respect to the groove portion 139, in a radial direction of the ball screw shaft 135, as will be described later. Specifically, the detent 137 is movable outward which is a direction away from the ball screw shaft 135 or inward which is a direction approaching the ball screw shaft 135. Incidentally, movement modes of the detent 137 include a case where the entire detent 137 moves and a case where a part of the detent 137 moves so that the detent 137 is inclined.

As illustrated in FIG. 12, the detent 137 has a substantially elliptical outer shape. Then, the groove portion 139 has an inner shape complementary to the outer shape of detent 137. Therefore, the groove portion 139 is formed as a substantially elliptical bottomed hole. In addition, a gap is set between the detent 137 and the groove portion 139. As an example, a gap ranging from 0.1 mm to 0.05 mm is set between a side face of the detent 137 and an inner face of the groove portion 139. Similarly, a gap may be set between the side face of the detent 137 and an inner face of the guide groove 138. Furthermore, an oil groove 137A through which a lubricant such as a food machine lubricating oil flows is formed on a top face of the detent 137. As an example, the lubricant is spray-applied to the detent 137. Then, as the ball screw nut 136 moves forward or backward, the lubricant spreads between the detent 137 and the guide groove 138.

Furthermore, as illustrated in FIG. 13, a storage portion 137B for the lubricant is formed on a bottom face of the detent 137. Specifically, the storage portion 137B is formed in the detent 137 at a position facing the groove portion 139. That is, the storage portion 137B is formed on the bottom face opposite to the top face, so as to oppose the guide groove 138. As an example, the storage portion 137B has a substantially elliptical shape. In addition, a length of the storage portion 137B along the longitudinal direction of the detent 137 is longer than that of the oil groove 137A.

The lubricant (as an example, a food machine lubricating oil conforming to the H1 standard registered in the National Sanitation Foundation International (NSF)) is applied to the storage portion 137B. The lubricant is stored in the storage portion 137B, and is supplied from the storage portion 137B to the gap between the detent 137 and the groove portion 139 when the detent 137 moves with respect to the groove portion 139. As an example, the lubricant applied to the storage portion 137B has a higher viscosity than the lubricant applied to the top face of the detent 137. Since the viscosity is high, the detent 137 adheres to the groove portion 139, so that the detent 137 can be prevented from falling off. Incidentally, instead of the storage portion 137B, the lubricant may be applied to a bottom of the groove portion 139. The lubricant applied in such a manner enters the inside of the storage portion 137B by pushing the detent 137 into the groove portion 139. Furthermore, when the storage portion storage portion 137B is not formed, the lubricant may be applied to the bottom face of the detent 137.

As an example, a method for manufacturing the actuator 130 described above includes steps described below. First, as a preparation step, the case 170 in which the guide grooves 138 for guiding the detents 137, respectively, are formed and the ball screw nut 136 in which the groove portions 139 are formed are prepared. As an example, the case 170 is made of aluminum and is subjected to alumite treatment. In addition, the presser pipe 131 is fixed to the ball screw nut 136. Then, as an application step, the lubricant is applied to at least one of the detents 137 or the groove portions 139. Here, excessive application of the lubricant can be prevented by applying the lubricant to the storage portions 137B of the detents 137. Subsequently, as an arrangement step, the detents 137 are arranged such that the detents 137 are partially located inside the groove portions 139, respectively. Then, the detents 137 are arranged such that the storage portions 137B oppose the groove portions 139. As an example, a material of the detents 137 is poly ether ether ketone (PEEK).

In addition, as a spray application step, the lubricant is spray-applied to the top faces of the detents 137. The spray application step may be performed before the arrangement step of the detents 137. Then, the drive mechanism DM is housed inside the case 170 such that the detents 137 are located between the guide grooves 138 and the groove portions 139, respectively. Specifically, the drive mechanism DM is inserted into the case 170 such that the detents 137 are partially located inside the guide grooves 138, respectively. Thereafter, the actuator 130 is manufactured by attaching a rear cap and a front cap, which are not illustrated, to the case 170. Incidentally, the detents 137 may be formed using a low friction resin (for example, iglidur manufactured by Igus k.k.). In addition, a slidable resin film may be attached to the detents 137 or the guide grooves 138. In such cases, the spray application step may be omitted.

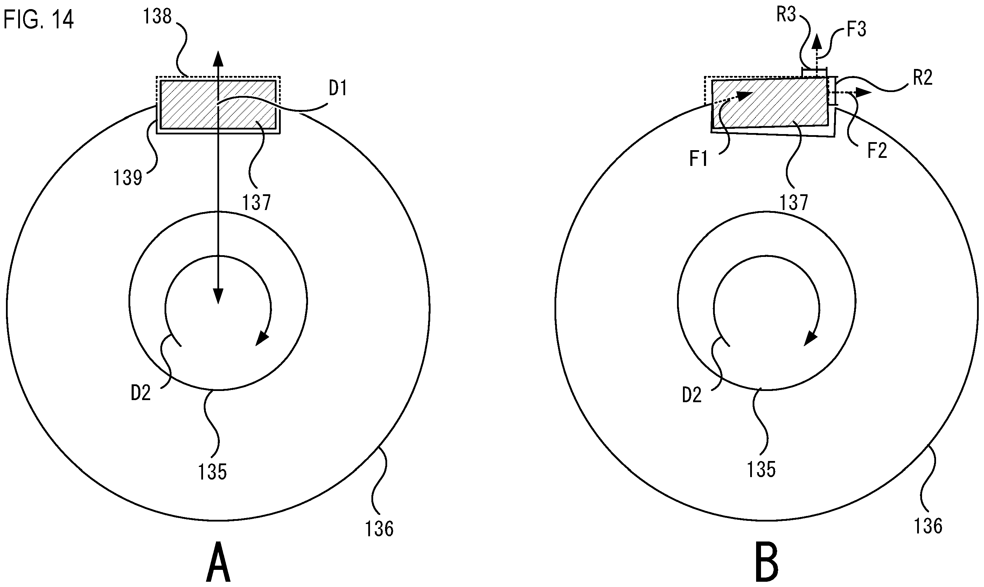

Next, movement of the detent 137 will be described with reference to FIG. 14. Incidentally, FIG. 14 is an explanatory view for describing the movement of the detent 137. Then, FIG. 14A illustrates the detent 137 before the movement, and FIG. 14B illustrates the detent 137 after the movement. In addition, FIG. 14 is a view schematically illustrating cross sections of the ball screw shaft 135, the ball screw nut 136, and the detent 137. However, only one detent 137 is illustrated, and illustrations of the other detents 137 are omitted. As described above, the detent 137 is movable in a radial direction D1 of the ball screw shaft 135 with respect to the groove portion 139. Accordingly, a force applied to the guide groove 138 and the case 170 via the detent 137 is dispersed. Therefore, damage to the case 170 can be suppressed even when a thickness of the case 170 is reduced for downsizing.

Specifically, as illustrated in FIG. 14A, the detent 137 is positioned between the guide groove 138 indicated by a dotted line and the groove portion 139 indicated by a solid line. Then, when the ball screw shaft 135 rotates in a rotation direction D2, the inner face of the groove portion 139 and the side face of the detent 137 abut on each other. Therefore, the detent 137 is pushed by the inner face of the groove portion 139, and one edge of the detent 137 abuts on an inner face of the guide groove 138. Accordingly, the rotation of the ball screw nut 136 is restricted. Then, the gap is set between the outer face of the detent 137 and the inner face of the groove portion 139, and a film is formed by the lubricant supplied from the storage portion 137B. Therefore, the detent 137 is in the state such as floating in the groove portion 139.

As a result, as illustrated in FIG. 14B, the detent 137 moves outward in the radial direction D1 by a force, indicated by an arrow F1, applied from the groove portion 139 to the detent 137. In addition, the detent 137 moves in a direction along the outer peripheral face of the ball screw nut 136 inside the groove portion 139. Then, the detent 137 is pushed against the guide groove 138 and takes an inclined posture. Therefore, the top face and the side face of the detent 137 are pushed against the guide groove 138. Accordingly, a force applied from the detent 137 to the guide groove 138 is dispersed as indicated by an arrow F2 and an arrow F3. In other words, the detent 137 and the guide groove 138 abut on each other at a plurality of places so that a contact area can be increased. Therefore, the force applied to the guide groove 138 in each region decreases.

In the example of FIG. 14B, the forces are applied from the detent 137 to the guide groove 138 in a region R2 and a region R3. Therefore, the force applied to each of the region R2 and the region R3 is dispersed and reduced. Accordingly, damage to the guide groove 138 and the case 170 can be suppressed. Furthermore, the force applied to the guide groove 138 can be further dispersed by arranging the plurality of detents 137. On the other hand, if the detent 137 does not move, the detent 137 and the guide groove 138 abut on each other only in the region R2. Therefore, the contact area is narrow, and the force applied to the guide groove 138 increases.

Incidentally, when the ball screw shaft 135 rotates in a direction opposite to the rotation direction D2, the detent 137 is inclined in the opposite direction. Specifically, one edge of the detent 137 moves inward in the radial direction D1 which is the direction approaching the ball screw shaft 135. In addition, the other edge of the detent 137 moves outward in the radial direction D1 which is the direction away from the ball screw shaft 135. Then, a top face and a side face of the other edge of the inclined detent 137 abut on the guide groove 138.

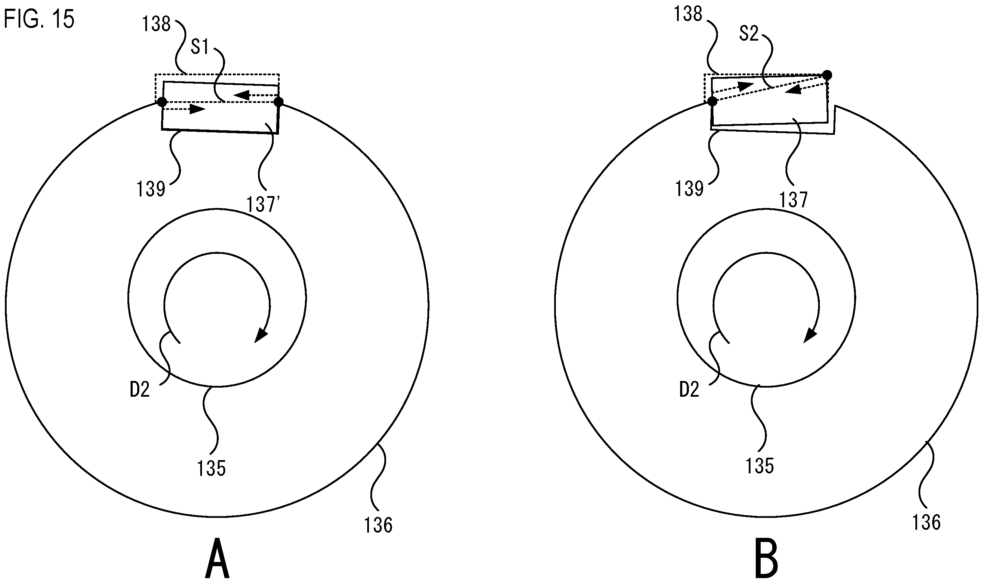

Subsequently, shearing of the detent 137 will be described with reference to FIG. 15. Incidentally, FIG. 15 is an explanatory view for describing the shearing of the detent 137. Then, FIG. 15A illustrates a detent 137' in a case of not moving, and FIG. 15B illustrates the detent 137 in a case of moving. In addition, FIG. 15 is a view schematically illustrating cross sections of the ball screw shaft 135, the ball screw nut 136, and the detent 137. However, only one detent 137 is illustrated, and illustrations of the other detents 137 are omitted.

As an example, the detent 137' illustrated in FIG. 15A is fixed to the groove portion 139 by adhesion. Then, when the ball screw shaft 135 rotates in the rotation direction D2, a force is applied to the detent 137' from an abutment region of a side face abutting on the groove portion 139 indicated by a solid line. Furthermore, a reaction force is applied to the detent 137' from an abutment region of a side face abutting on the guide groove 138 indicated by a dotted line. As a result, shear stress is applied to the detent 137' in a cross section S1 passing through the two abutment regions.

In addition, when the ball screw shaft 135 rotates in the rotation direction D2, a force is applied to the detent 137, in the case of moving illustrated in FIG. 15B, from an abutment region of the side face abutting on the groove portion 139 indicated by a solid line. Then, a reaction force is applied to the detent 137 from an abutment region of a corner abutting on the guide groove 138 indicated by a dotted line. As a result, shear stress is applied to the detent 137 in a cross section S2 passing through the two abutment regions.

Here, the cross section S1 of the detent 137' extends from the side face to the side face, whereas the cross section S2 of the detent 137 extends from the side face to the corner. Therefore, a cross-sectional area of the cross section S2 is larger than that of the cross section S1. As a result, the shear stress applied to the detent 137 is smaller than that of the detent 137'. Accordingly, it is possible to suppress the detent 137 from being sheared off along the cross section S2.

According to the detent 137 described above, the detent 137 is not fixed to the groove portion 139. Therefore, the detent 137 is movable with respect to the groove portion 139 in the direction away from the groove portion 139 and in the direction approaching the groove portion 139. As a result, a force can be received in a plurality of regions of the guide groove 138, and thus, the force applied to the guide groove 138 is dispersed, and the damage to the case 170 can be suppressed even if the case 170 having a smaller thickness and a lower strength is used. Therefore, the thin case 170 can be used, the size of the injection device 100 can be reduced, and the weight of the injection device 100 can be reduced. Furthermore, the detent 137 can be suppressed from being sheared off.

Hitherto, the present invention has been described with reference to the embodiments, but the present invention is not limited to the above embodiments. Inventions modified within a range without contradictory to the present invention and inventions equivalent to the present invention are also included in the present invention. In addition, the embodiments and the modifications and technical means included in the embodiments or the modifications can be appropriately combined within a range not contradictory to the present invention.

For example, the injection device 100 can be connected to an imaging device in a wired or wireless manner. Then, various types of data are transmitted and received between the imaging device and the injection device 100 when the liquid medicine is injected and when an image is captured. In such a case, for example, imaging conditions may be set or displayed in the injection device 100, and the injection conditions may be set or displayed in the imaging device. Examples of such an imaging device include various medical imaging devices such as a magnetic resonance imaging (MRI) device, a computed tomography (CT) device, an angiography imaging device, a positron emission tomography (PET) device, a single photon emission computed tomography (SPECT) device, a CT angiography device, an MR angiography device, an ultrasonic diagnostic device, and a blood vessel imaging device.

In addition, the injection device 100 can cause information regarding an injection result (for example, injection history) to be transmitted to and stored in external storage devices such as a radiology information system (RIS), a picture archiving and communication systems (PACS), and a hospital information system (HIS) via a network.

In addition, the syringes S to be mounted on the injection device 100 may be either the syringes S filled with the liquid medicines or the empty syringes S that are not filled with any liquid medicine. The syringes S filled with the liquid medicines include a prefilled syringe filled with the liquid medicine in advance, the syringe S obtained by the operator filling the empty syringe S with the liquid medicine by an aspirator or a filling machine, the syringe S obtained by the operator manually filling the empty syringe S with the liquid medicine, and the like.

In addition, when the empty syringe S not filled with the liquid medicine is mounted on the injection device 100, the operator can fill the liquid medicine in the syringe S by the injection device 100, the aspirator, or the filling machine. Furthermore, the syringe S can be provided with a data carrier such as a radio frequency identifier (RFID) or a barcode. Information regarding the filled liquid medicine and the like are recorded in the data carrier. Then, the injection device 100 includes a reading device that reads the recorded information from the data carrier, and can control an injection pressure of the liquid medicine and the like.

In addition, remote operation devices such as a foot switch and a hand switch may be connected to the injection device 100 in a wired or wireless manner. The operator can operate the injection device 100 by operating the remote operation devices instead of the operation buttons 143.

In addition, instead of the ball screw shaft and the ball screw nut, a trapezoidal screw shaft and a trapezoidal screw nut, which is an example of the sliding member, can be used as the drive mechanism DM. However, no ball is used when the trapezoidal screw shaft and the trapezoidal screw nut are used. Therefore, the efficiency of converting rotational motion into linear motion decreases due to frictional resistance. Furthermore, due to the frictional resistance, a straight traveling speed of the presser 115 decreases, and detection accuracy of torque applied to the presser 115 decreases. Therefore, by using the ball screw shaft and the ball screw nut, the decrease in the straight traveling speed and the detection accuracy of the torque can be suppressed.

In addition, the transmission mechanism 180 may include an idle gear that meshes with the driving gear 181 and the driven gear 182 instead of the timing belt 183. Furthermore, a part of the lower cover 142 may be directly or indirectly fixed to at least one of the syringe holder 110, the front plate 171, the device support 114, the actuator 130, or the rear plate 175. In addition, a part of the upper cover 141 may be directly or indirectly fixed to at least one of the syringe holder 110, the front plate 171, the device support 114, or the rear plate 175.

Some or all of the above embodiments can also be described as the following in Supplements, but are not limited to the following.

### (Supplement 1)

An injection device comprising:
a syringe holder on which a syringe filled with a liquid medicine is mounted;
a presser that pushes out the liquid medicine from the mounted syringe;
an actuator including a case that houses a drive mechanism moving the presser forward or backward; and
a first cover and a second cover that house the actuator, wherein
at least one of the first cover or the second cover is fixed to the case.

### (Supplement 2)

The injection device according to Supplement 1, wherein the case is provided with a fixing portion to which at least one of the first cover or the second cover is fixed.

### (Supplement 3)

The injection device according to Supplement 2, wherein
a plurality of the actuators are included, and
the fixing portion is arranged at a position by avoiding a space between the plurality of actuators.

### (Supplement 4)

The injection device according to any one of Supplements 1 to 3, wherein
a plurality of the actuators are arranged, and
the cases of the plurality of actuators are coupled by one plate member.

### (Supplement 5)

The injection device according to any one of Supplements 1 to 4, further comprising
a coupling portion that couples a device support of the injection device and the case, wherein
the coupling portion is fixed to the case.

### (Supplement 6)

The injection device according to any one of Supplements 1 to 5, further comprising:
a motor; and
a transmission mechanism that transmits rotation from the motor to the actuator, wherein
the actuator includes a feed screw nut and a feed screw shaft.

### (Supplement 7)

The injection device according to Supplement 6, wherein
the transmission mechanism includes a rotation member that transmits the rotation from the motor to the actuator, and
a magnet for a magnetic encoder is arranged on the rotation member on a side facing the actuator.

### (Supplement 8)

The injection device according to any one of Supplements 1 to 5, wherein
the drive mechanism includes a feed screw shaft and a sliding member that moves along the feed screw shaft with rotation of the feed screw shaft,
the sliding member includes a plurality of detents,
a guide groove guiding each of the detents is formed in the case, and
each of the detents is arranged in a groove portion formed in the sliding member and is movable in a radial direction of the feed screw shaft with respect to the groove portion.

### (Supplement 9)

An injection device that injects a liquid medicine, comprising:
a presser that pushes out the liquid medicine; and
an actuator including a drive mechanism that moves the presser forward or backward and a case that houses the drive mechanism, wherein
the drive mechanism includes a feed screw shaft and a sliding member that moves along the feed screw shaft with rotation of the feed screw shaft,
the sliding member includes a plurality of detents,
a guide groove guiding each of the detents is formed in the case, and
each of the detents is arranged in a groove portion formed in the sliding member and is movable in a radial direction of the feed screw shaft with respect to the groove portion.

### (Supplement 10)

The injection device according to Supplement 8 or 9, wherein
a storage portion for a lubricant is formed in each of the detents, and
the storage portion is formed in each of the detents at a position facing the groove portion.

### (Supplement 11)

The injection device according to any one of Supplements 8 to 10, wherein a gap is set between each of the detents and the groove portion.

### (Supplement 12)

A method for manufacturing an actuator, the actuator including a drive mechanism including a feed screw shaft and a sliding member that moves along the feed screw shaft with rotation of the feed screw shaft, and a case that houses the drive mechanism, the sliding member including a plurality of detents, the method comprising:
preparing the case in which a guide groove guiding each of the detents is formed;
preparing the sliding member in which a groove portion is formed;
applying a lubricant to at least either each of the detents or the groove portion;
arranging each of the detents in the groove portion; and
housing the drive mechanism inside the case in such a manner that each of the detents is located between the guide groove and the groove portion.

The application claims priority from Japanese Patent Application No. 2022-092096 filed on June 7, 2022, the entire contents of which are incorporated herein by reference.

### Reference Signs List

- 100: Injection device
- 110: Syringe holder
- 114: Device support
- 115: Presser
- 130: Actuator
- 132: Motor
- 135: Ball screw shaft (feed screw shaft)
- 136: Ball screw nut (sliding member)
- 137: Detent
- 137B: Storage portion
- 138: Guide groove
- 139: Groove portion
- 141: Upper cover (first cover)
- 142: Lower cover (second cover)
- 170: Case
- 170A: Fixing portion
- 175: Rear plate (plate member)
- 180: Transmission mechanism
- 182: Driven gear (rotation member)
- 182A: Magnet
- 214C: Connection member (coupling portion)
- D1: Radial direction
- DM: Drive mechanism
- S: Syringe

## Claims

1. An injection device comprising:
a syringe holder on which a syringe filled with a liquid medicine is mounted;
a presser that pushes out the liquid medicine from the mounted syringe;
an actuator including a case that houses a drive mechanism moving the presser forward or backward; and
a first cover and a second cover that house the actuator, wherein
at least one of the first cover or the second cover is fixed to the case.

2. The injection device according to claim 1, wherein the case is provided with a fixing portion to which at least one of the first cover or the second cover is fixed.

3. The injection device according to claim 2, wherein
a plurality of the actuators are included, and
the fixing portion is arranged at a position by avoiding a space between the plurality of actuators.

4. The injection device according to any one of claims 1 to 3, wherein
a plurality of the actuators are arranged, and
the cases of the plurality of actuators are coupled by one plate member.

5. The injection device according to any one of claims 1 to 3, further comprising
a coupling portion that couples a device support of the injection device and the case, wherein
the coupling portion is fixed to the case.

6. The injection device according to any one of claims 1 to 3, further comprising:
a motor; and
a transmission mechanism that transmits rotation from the motor to the actuator, wherein
the actuator includes a feed screw nut and a feed screw shaft.

7. The injection device according to claim 6, wherein
the transmission mechanism includes a rotation member that transmits the rotation from the motor to the actuator, and
a magnet for a magnetic encoder is arranged on the rotation member on a side facing the actuator.

8. The injection device according to any one of claims 1 to 3, wherein
the drive mechanism includes a feed screw shaft and a sliding member that moves along the feed screw shaft with rotation of the feed screw shaft,
the sliding member includes a plurality of detents,
a guide groove guiding each of the detents is formed in the case, and
each of the detents is arranged in a groove portion formed in the sliding member and is movable in a radial direction of the feed screw shaft with respect to the groove portion.

9. An injection device that injects a liquid medicine, comprising:
a presser that pushes out the liquid medicine; and
an actuator including a drive mechanism that moves the presser forward or backward and a case that houses the drive mechanism, wherein
the drive mechanism includes a feed screw shaft and a sliding member that moves along the feed screw shaft with rotation of the feed screw shaft,
the sliding member includes a plurality of detents,
a guide groove guiding each of the detents is formed in the case, and
each of the detents is arranged in a groove portion formed in the sliding member and is movable in a radial direction of the feed screw shaft with respect to the groove portion.

10. The injection device according to claim 9, wherein
a storage portion for a lubricant is formed in each of the detents, and
the storage portion is formed in each of the detents at a position facing the groove portion.

11. The injection device according to claim 9 or 10, wherein a gap is set between each of the detents and the groove portion.

12. A method for manufacturing an actuator, the actuator including a drive mechanism including a feed screw shaft and a sliding member that moves along the feed screw shaft with rotation of the feed screw shaft, and a case that houses the drive mechanism, the sliding member including a plurality of detents, the method comprising:
preparing the case in which a guide groove guiding each of the detents is formed;
preparing the sliding member in which a groove portion is formed;
applying a lubricant to at least either each of the detents or the groove portion;
arranging each of the detents in the groove portion; and
housing the drive mechanism inside the case in such a manner that each of the detents is located between the guide groove and the groove portion.
